(19) Europäisches Patentamt / European Patent Office / Office européen des brevets

(11) **EP 4 602 925 A1**

(12) **EUROPEAN PATENT APPLICATION**

(43) Date of publication:
**20.08.2025 Bulletin 2025/34**

(21) Application number: **25155605.6**

(22) Date of filing: **03.02.2025**

(51) International Patent Classification (IPC):
***A23K 20/111*** *(2016.01)* ***A23K 20/132*** *(2016.01)*
***A23K 20/158*** *(2016.01)* ***A23L 29/00*** *(2016.01)*
***A61K 31/06*** *(2006.01)*

(52) Cooperative Patent Classification (CPC):
**A23L 29/045; A23K 20/111; A23K 20/132; A23K 20/158; A23L 29/04; A61K 31/06**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **19.02.2024 IT 202400003442**

(71) Applicants:
- **A.W.P. S.P.A.**
  **41051 Castelnuovo Rangone (MO) (IT)**
- **Cerchiari, Emilio**
  **8055 Graz (AT)**

(72) Inventors:
- **Cristofori, Paolo**
  **42123 Reggio Emilia (IT)**
- **Cerchiari, Emilio**
  **8055 Graz (AT)**

(74) Representative: **Corradini, Cesare et al**
**Ing. C. Corradini & C. S.r.l.**
**Via Dante Alighieri, 4**
**42121 Reggio Emilia (IT)**

(54) **MIXTURE**

(57) A mixture is described comprising: at least one organic or inorganic acid with bactericidal and/or bacteriostatic properties; at least one essential oil with anti-inflammatory and antioxidant properties; at least one fatty acid; at least one plant extract; at least one carrier substance.

EP 4 602 925 A1

## Description

### TECHNICAL FIELD

The present invention relates to the field of human and/or animal nutrition/care, e.g. for farm or domestic animals/pets, in particular the present invention relates to a nutritional and/or pharmacological mixture with bacteriostatic activity, particularly suitable for the eradication of bacterial resistance mechanisms to antibiotics and other pharmacological additives, to be used as a food additive and/or nutraceutical for human and/or farm or domestic animal foodstuffs and the related food.

### PRIOR ART

Bacterial resistance to antibiotics is commonly known, particularly in the fields of pharmacology and nutrition (animal and human), and represents one of the most important and dreaded challenges for the future of humans and animals.

Bacterial resistance, or "antimicrobial" resistance, occurs when germs such as bacteria, parasites and fungi develop the ability to defeat drugs designed to kill them, so much so that some types of infection can become so resistant that they are essentially impossible to treat.

Consequently, antimicrobial resistance represents a serious threat to global public health, as it is linked to an average of at least 1.27 million deaths per year and, in 2019, was associated with approximately 5 million deaths. Every year, in the US alone, more than 2.8 million antimicrobial-resistant infections occur, resulting in approximately 35 thousand deaths. Similarly, an estimated 35,000 deaths associated with antimicrobial-resistant infections also occurred in Europe, according to a report published in November 2022. Furthermore, this estimate looks at the five-year period 2016-2020 and shows an increase compared to previous estimates.

The impact of antimicrobial resistance on general health is comparable to that of flu, tuberculosis and HIV/AIDS combined.

According to a recent report by the UK government, antibiotic resistance is estimated to cause 500 million premature deaths by 2050, with losses of up to 100 trillion to the global economy.

A need perceived in the industry is, therefore, to make available a composition capable of obviating such bacterial or antimicrobial resistance, i.e. essentially a bactericidal and/or bacteriostatic agent capable of eradicating the defence mechanisms of pathogenic microorganisms, especially those with proven resistance to antibiotics, so as to restore the efficacy of said antibiotics and other pharmacological additives.

An object of the present invention is to solve these and other needs of the prior art, with a simple, rational and cost-effective solution.

Such object is achieved by the features of the invention reported in the independent claim. The dependent claims outline preferred and/or particularly advantageous aspects of the invention.

### DISCLOSURE OF THE INVENTION

The invention makes a (nutritional and/or pharmaceutical and/or nutraceutical) mixture available.

The mixture is for example used as a food additive in human food and/or food/feed for farm or domestic animals.

In the context of the present invention, human food means any food and/or any nutritional substance, whether in solid, powder, liquid or gel form or otherwise.

In the context of the present invention, feed or food for animals means, by way of nonlimiting example, a feed or food for pigs, fish, poultry species, herbivorous species, such as cattle or sheep or horses or the like, at any stage of their growth and/or a feed or food for any pet, such as dogs, cats, rabbits or the like.

The animal feed/food of the present invention is, preferably, a dry feed/food in the form of pellets, cubes, granules or powders or wet or the like that has nutritional properties for the animal.

The mixture (additive), preferably, is a composition/mixture in solid/powder or liquid form. The mixture, preferably, comprises:

- at least one organic or inorganic acid with bactericidal and/or bacteriostatic properties,
- at least one essential oil with anti-inflammatory and antioxidant properties,
- at least one fatty acid,
- at least one plant extract,
- at least one carrier substance.

The mixture may further comprise at least one mineral salt, e.g. advantageously a plurality of said mineral salts.

A mixture capable of meeting the needs of the prior art is thus made available, i.e. an agent capable of suppressing the defence systems of pathogenic micro-organisms resistant to antibiotics, so as to make the bacteria sensitive to the drugs again and, therefore, such that antibiotics and any other pharmacological additives become effective again.

Advantageously, all components of the mixture have bacteriostatic or bactericidal properties, thus configured to reduce bacterial defences and consequently limit drug resistance.

In addition, the combination of these components enhances the biological potential of the individual ingredients.

According to a first aspect of the invention, said at least one organic or inorganic acid may be selected from the group consisting of: citric acid, sorbic acid, malic acid, orthophosphoric acid, fumaric acid, benzoic acid, acetic acid, butyric acid, formic acid and lactic acid, in free form and/or as salts.

According to another aspect of the invention, said at least one essential oil can be selected from the group consisting of alcohols, aldehydes, ketones, esters, phenols, sesquiterpenes and terpenes.

Again, said at least one essential oil can be obtained from a pure plant extract identical to the natural one.

According to another aspect of the invention, said at least one fatty acid may be a medium chain fatty acid in free form (MCFA) and/or as a monoglyceride.

In particular, said medium chain fatty acid (MCFA) can be defined as a fatty acid saturated with hydrocarbon chains with 6 or 12 carbon atoms.

Furthermore, the corresponding monoglyceride derivative of said fatty acid may be a single-chain lipid amphiphile.

Examples of medium-chain fatty acids (MCFA) and monoglycerides according to the invention are shown in the table below:

| | Name of the compound | Chemical structure | Molecular weight Dalton | Melting point, °C | Visual appearance | Odour |
|---|---|---|---|---|---|---|
| **Fatty acids** | Caproic acid (C6H12O2) | 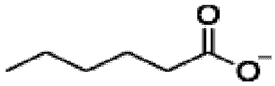 | 116.2 | -3.4 | Oily liquid, colourless | Strong |
| | Caprylic acid (C8H16O2) | 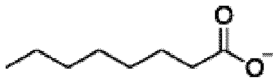 | 144.2 | 16.5 | Oily liquid, colourless | Moderate |
| | Capric acid (C10H20O2) | 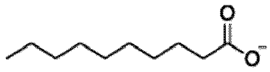 | 172.3 | 31.6 | White crystalline powder | Light |
| | Lauric acid (C12H24O2) | 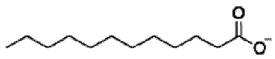 | 200.3 | 43.8 | White powder | Slight |
| **Monoglycerides** | Glycerol monocaproate / Monocaproin (C9H18O4) | 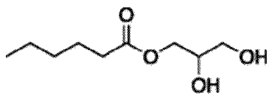 | 190.2 | 19.4 | Colourless liquid | Slight |
| | Glycerol monocaprylate / Monocaprylin (C11H22O4) | 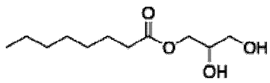 | 218.3 | 35.6 | White powder | Slight |
| | Glycerol monocaprate / Monocaprin (C13H26O4) | 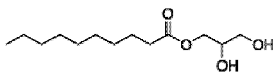 | 246.3 | 51.4 | White powder | Slight |
| | Glycerol monolaurate / Monolaurin (C15H30O4) | 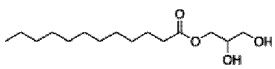 | 274.4 | 62.5 | Granular white powder | Slight |

According to a further aspect of the invention, the plant extract may be purified lignin.

According to a further aspect of the invention, the carrier substance may consist of at least one mineral selected from: zeolites, bentonites, silicates, calcium salts, potassium salts and sodium chloride in the case of a dry mixture, and oils in the case of a liquid mixture.

Advantageously, the invention makes available a mixture capable of mitigating the pathogenicity of pathogenic microorganisms and breaking down the biological defences of said pathogenic microorganisms as well as the Quorum Sensing thereof.

BEST WAY TO ACTUATE THE INVENTION

The invention relates to a mixture, particularly a nutritional and/or pharmacological (in the human and/or veterinary field) and/or nutraceutical mixture, which has bacteriostatic properties and in that sense is capable of reducing bacterial defences and eradicating the mechanisms of resistance to antibiotics in humans and/or animals, so as to restore the efficacy of said antibiotics and other pharmacological additives (in the human and/or veterinary field).

A (main) purpose of the mixture according to the invention is to inhibit the defences of microorganisms exhibiting drug resistance against antibiotics.

The mixture in fact acts on the membranes of the pathogen and also on protein and energy synthesis.

In addition, the mixture inhibits biochemical communication - known as quorum sensing *(QS)* - which activates pathogenesis through complex biochemical activity.

Quorum sensing (QS), discovered in 1970, is a bacterial communication mechanism using diffusible molecules known as self-inducers, which plays an important role in the induction of gene expression to control cellular behaviour such as:

- ➢ secretion of virulent factors,
- ➢ sporulation
- ➢ production of secreted proteolytic enzymes
- ➢ survival to antimicrobial agents (antibiotic multi resistance- AMR)
- ➢ biofilm development

As is well known, survival to antimicrobial agents and biofilm development are a very strong and effective biological coalition to induce pathogenesis.

The mixture according to the present invention is preferably used simultaneously with an antibiotic for the duration of the treatment in order to exploit the synergism between the two components.

6-In particular, the mixture can be used together with any antibiotic. Inhibiting the defences of the drug-resistant pathogenic micro-organism allows the antibiotic to be a potent biocide again, effectively wiping out the drug resistance (which had previously been developed by the organism).

For example, in the case of antibiotic therapy against pathogenic but non-resistant microorganisms, the mixture, by inhibiting the biological potential of the pathogens, still promotes the biocidal activity of the antibiotic. In fact, the patient achieves full recovery more quickly (compared to the use of antibiotics alone). This factor also reduces antibiotic consumption in a general sense.

The mixture first comprises at least one organic or inorganic acid. In particular, said organic or inorganic acid has bactericidal and/or bacteriostatic properties.

For example, the organic or inorganic acid can be selected from the group consisting of citric acid, sorbic acid, malic acid, orthophosphoric acid, fumaric acid, benzoic acid, acetic acid, butyric acid, formic acid and lactic acid.

Additionally, the acid can be selected that group in free form and/or as salts.

Said at least one organic or inorganic acid may be present in the mixture in a weight percentage (wt%) of the total weight of the mixture comprised between 1 % and 80 %, e.g. between 18 % and 46 %, more precisely see the examples below.

In any case, it is not excluded that the mixture may comprise a plurality of organic or inorganic acids, all of which have bactericidal and/or bacteriostatic properties and are selected from the above-mentioned group.

The mixture also comprises at least one essential oil. In particular, said essential oil has anti-inflammatory and antioxidant properties.

The essential oil can be selected from the group consisting of alcohols, aldehydes, ketones, esters, phenols, sesquiterpenes and terpenes.

Additionally, said essential oil can be obtained from a pure plant extract identical to the natural one.

Said essential oil may be present in the mixture in a weight percentage (wt%) of the total weight of the mixture comprised between 0.1 % and 98 %, e.g. between 7 % and 40 %, more precisely see the examples below.

It is also not excluded that the mixture may comprise a plurality of said essential oils, distinct from each other and,

for example, each selected from the group of essential oils listed above.

The mixture then comprises a fatty acid, e.g. a medium chain fatty acid (MCFA). In particular, said fatty acid may be present in free form and/or as a monoglyceride. Preferably, the corresponding monoglyceride derivative of said fatty acid is a single-chain lipid amphiphile.

Preferably, said fatty acid is present in the mixture in a weight percentage (wt%) of the total weight of the mixture comprised between 1 % and 90 %, e.g. comprised between 5 % and 25 %, more preferably see the examples below.

Additionally, the mixture comprises a plant extract.

Particularly, said plant extract may be purified lignin, however, it is not excluded that it may be another extract that is particularly useful for the intended purposes.

It is also not excluded that the mixture may comprise a plurality of said plant extracts, different from each other.

The plant extract may be present in the mixture in a weight percentage (wt%) of the total weight of the mixture comprised between 0.1 % and 78 %, e.g. it may be comprised between 2 % and 5 %, more precisely see the examples below.

Again, the mixture comprises a carrier substance.

In detail, if the mixture is a dry mix, said carrier substance may consist of at least one mineral, e.g. selected from the group consisting of: zeolites, bentonites, silicates, calcium salts, potassium salts and sodium chloride. In the case of a liquid mixture, the carrier protein may consist of an oil.

Said carrier substance may be present in the mixture in a weight percentage (wt%) of the total weight of the mixture comprised between 5 % and 85 %, e.g. comprised between 32 % and 40 %, more precisely see the examples below.

Additionally, the mixture may comprise at least one mineral salt, e.g. a plurality of said mineral salts.

For example, said at least one mineral salt can be selected from the group consisting of: Zinc (in sulphate, carbonate and oxide salt form), Iron (in sulphate, carbonate, gluconate salt form) Copper (in sulphate, carbonate salt form) Manganese (in sulphate, carbonate, chloride salt form) also in chelate form, and magnesium citrate or mixtures thereof. Additionally, said at least one mineral salt may be present in the mixture in a weight percentage (wt%) of the total weight of the mixture comprised between 0.1% and 10%, more precisely see the examples below.

A first example of a mixture according to the invention, particularly a powder mixture, may comprise the following components, by weight percentage (wt%) with respect to the total weight of the mixture:

- essential oils, 20 %
- fatty acids, e.g. MCFA, 20 %
- fumaric acid, 20 %
- purified lignin, 5 %
- carrier substance, particularly a mineral, 35 %.

According to said first example, the components of the mixture can be mixed in a steel mixer and then added to the protective mineral matrix.

A second example of a mixture according to the invention, particularly a liquid mixture, may comprise the following components, by weight percentage (wt%) with respect to the total weight of the mixture:

- essential oils, 40 %
- fatty acids, e.g. MCFA, 7 %
- monoglycerides, 27 %
- citric acid, 18 %
- oily carrier substance, 35 %.

According to said second example, the components of the mixture can be mixed in a steel mixer and then added to the oily carrier substance.

A third example of a mixture according to the invention, particularly a powder mixture, may comprise the following components, by weight percentage (wt%) with respect to the total weight of the mixture:

- essential oils, 10 %
- fatty acids, e.g. MCFA, 10 %
- monoglycerides, 20 %
- formate (i.e. formic acid), 18 %
- purified lignin, 2 %
- carrier substance, particularly a mineral, 32 %.

According to said third example, the components of the mixture can be mixed in a steel mixer and then added to the oily carrier substance.

A fourth example of a mixture according to the invention, particularly a liquid mixture, may comprise the following components, by weight percentage (wt%) with respect to the total weight of the mixture:

- essential oils, 10 %
- fatty acids, e.g. MCFA, 10 %
- monoglycerides, 20 %
- benzoic acid, 20 %
- oily carrier substance, 40 %.

According to said fourth example, the components of the mixture can be mixed in a steel mixer and then added to the oily carrier substance.

A fifth example of a mixture according to the invention, particularly a powder mixture, may comprise the following components, by weight percentage (wt%) with respect to the total weight of the mixture:

- essential oils, 7 %
- fatty acids, e.g. MCFA, 8 %
- monoglycerides, 5 %
- benzoic acid, 20 %
- fumaric acid, 17 %
- formate (i.e. formic acid), 11 %
- butyrate (i.e. butyric acid), 8 %
- carrier substance, particularly a mineral, 32 %.

According to said fifth example, the components of the mixture can be mixed in a steel mixer and then added to the oily carrier substance.

A sixth example of a mixture according to the invention, particularly a liquid mixture, may comprise the following components, by weight percentage (wt%) with respect to the total weight of the mixture:

- essential oils, 20 %
- fatty acids, e.g. MCFA, 20 %
- monoglycerides, 30 %
- acetic acid, 10 %
- lactic acid, 10 %
- oily carrier substance, 40 %.

According to said sixth example, the components of the mixture can be mixed in a steel mixer and then added to the oily carrier substance.

A seventh example of a mixture according to the invention may comprise the following components, by weight percentage (wt%) of the total weight of the mixture:

- essential oils, 20 %
- fatty acids, e.g. MCFA, 7 %
- monoglycerides, 20 %
- citric acid, 18 %
- zinc sulphate, 0.5%
- magnesium citrate, 8.5 %
- carrier substance, particularly a mineral, 26 %.

According to said seventh example, the components of the mixture can be mixed in a steel mixer and then added to the oily carrier substance.

**<u>Experimental tests demonstrating synergism between the mixture according to the present invention (called TEST mixture) and Methicillin vs antibiotic-resistant Staphylococcus aureus (MRSA)</u>**

The experimental tests are divided into two steps:

- **Step 1:** evaluation of the antimicrobial activity of the **TEST** mixture and parallel methicillin against 2 strains of S. aureus with different antibiotic sensitivity.

In particular, the following strains were selected: **DSM 799,** standard strain and used here as a negative control because it is sensitive to methicillin.
**DSM 46320,** MRSA reference strain. At this stage, the antimicrobial synergism between the mixture and methicillin against the MRSA strain was also investigated, in order to identify the dose that would maximise the antibiotic effect.

- **Step 2:** evaluation of the ability of the **TEST** mixture to inhibit and eradicate the biofilm produced by S. aureus MRSA.

<u>**Step 1**</u>

Antimicrobial activity in "Step 1" was assessed in vitro, according to the CLSI (Clinical and Laboratory Standards Institute) protocol defined as the "broth microdilution method", suitably modified for the nutritional and growth requirements of the S. aureus strains under investigation (Table 1).

| | **Strains** | **Inoculum (CFU/ml)** | **Medium** | **Temperature (°C)** | **Incubation time (h)** |
|---|---|---|---|---|---|
| ***S. Aureus*** | DSM 799 (standard) | 10^5 | Muller Hinton | 37 | 20 |
| | DSM 46320 (methicillin-resistant) | | | | |

Table 1

In this test, carried out in 96-well microplates, the growth of microorganisms was tested at 24 different concentrations of the 2 compounds (test and methicillin) in order to determine their minimum inhibitory concentration (MIC).

**Control of antimicrobial activity - in Step 1**

Antimicrobial synergism in "Step 1" was assessed *in vitro* according to the protocol defined as the "broth microdilution checkerboard method", which allows the inhibitory effect of two agents to be assessed simultaneously by crossing serial dilutions thereof. For the **TEST** and methicillin, the fractional inhibitory concentration (FIC) was first calculated, i.e. the ratio between the MIC of the compound in combination and the MIC of the compound alone. This figure is considered reliable only in cases where the MIC of the compound in combination in the matrix is at least two dilutions lower than the MIC of the compound alone.
Subsequently, the sum of the FICs of the two compounds (**TEST** mixture and methicillin), i.e. the fractional inhibitory concentration index (FICI), was calculated according to the formula:

$$\text{F: } FICI = \frac{MIC\ ingredient\ A\ in\ combination}{MIC\ ingredient\ A\ alone} + \frac{MIC\ ingredient\ B\ in\ combination}{MIC\ ingredient\ B\ alone}$$

A: **TEST** mixture
B: **METHICILLIN**

The association is considered synergistic when the FICI is less than 1.

**Anti-biofilm activity in "Step 2"**

This activity was evaluated *in vitro* by means of adhesion tests conducted in 96-well microplates under the growth

conditions shown in Table 2, which allow the formation of S. aureus MRSA biofilms.

Table 2

| Strains | Inoculum (CFU/ml) | Medium | Temperature (°C) | Incubation time (h) |
|---|---|---|---|---|
| *S. aureus* DSM 46320 (methicillin-resistant) | 10^5 | TSA | 37 | 24 |

Specifically, to assess the ability of the **TEST** mixture to inhibit biofilm formation, the growth of microorganisms occurred in the presence of 8 different concentrations of the product. A positive control for growth and biofilm formation and a sterility control were also included, as in the test for the determination of **MIC.**

In both tests, the presence of biofilm in the wells was evaluated, after removal of the medium, by staining with Crystal Violet (0.1%) and spectrophotometrically reading the absorbance at 595 nm.

## Results

Evaluation of the MIC of the individual **TEST** mixture and individual for Methicillin against the 2 strains of S. aureus under investigation.

| **S. Aureus** | **Strains** | **MIC** | |
|---|---|---|---|
| | | **TEST mixture** (μg/mL) | **Methicillin** (μg/mL) |
| | DSM 799 (standard) | 3.1 | 0.5 |
| | DSM 46320 (methicillin-resistant) | 390.6 | 32.0 |

The antibiotic increase when used alone against methicillin-resistant Staphylococcus aureus to achieve MIC is very high. In the case of standard non-resistant Staphylococcus aureus, 0.5 μg/mL is sufficient to reach the MIC, whereas in the case of resistant Staphylococcus aureus, 32 μg/mL is required, a 64-fold increase.

The detection of the values for the **TEST** mixture also shows an important increase in quantity for achieving the MIC compared to use for non-resistant Staphylococcus aureus, 3.1 μg/mL, and resistant Staphylococcus aureus, 390.6 μg/mL, a 126-fold increase.

**Figure 1: Schematic representation of the "checkerboard method" for evaluating the antimicrobial synergism of the TEST mixture together with methicillin against the MRSA strain Staphylococcus aureus.**

Subsequently, the antimicrobial synergism between the **TEST** mixture product and Methicillin, on the MRSA strain (DSM 46320), was evaluated by crossing serial dilutions of the two compounds according to the "checkerboard method".

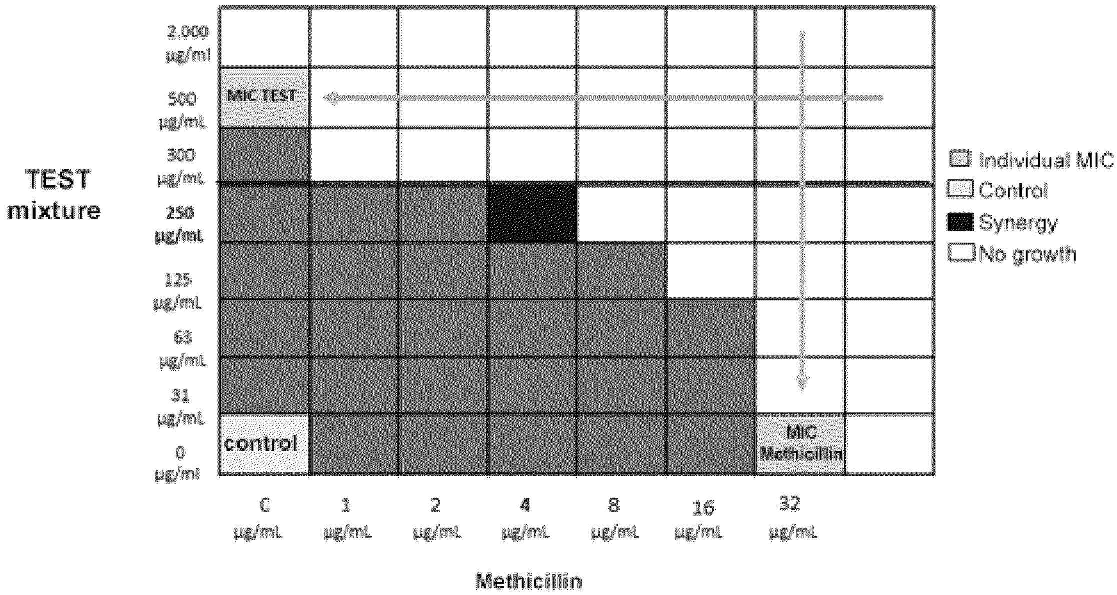


(Figure 1)

As shown in Figure 1, antimicrobial synergism was observed in the following concentration pair:

- 250 µg/mL **TEST** mixture and 4 µg/mL Methicillin

$$FICI = \frac{250\ \mu g/mL}{500\ \mu g/mL} + \frac{4\ \mu g/mL}{32\ \mu g/mL} = 0.625$$

Mathematical evaluation with FICI less than 1 displays strong synergistic activity. The amount of methicillin in combination with the **TEST** mixture required to achieve the **MIC** value is 8 times lower than the antibiotic used alone. The same applies to the **TEST** mixture with a 65% reduction.

After evaluating the antimicrobial activity of the **TEST** mixture on the planktonic (non-adherent) form, the activity on the S. aureus MRSA biofilm was examined in "Step 2".

**Figure 2:** Absorbance values at 595 nm after Crystal Violet staining of the existing biofilm formed by the MRSA strain in the presence of different **TEST** mixture concentrations in triplicate.

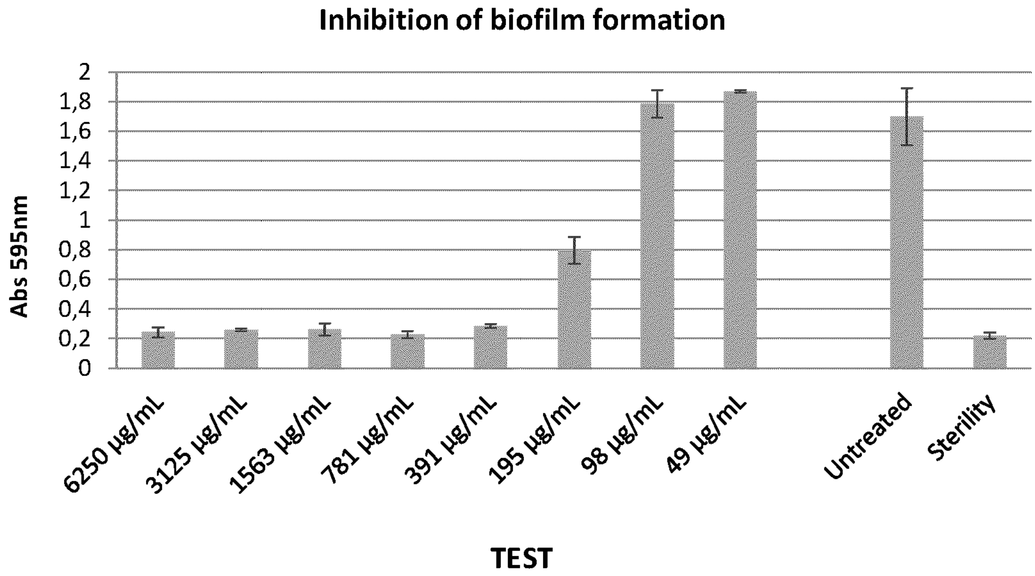


(Figure 2)

As shown in Figure 2, the **TEST** mixture totally inhibited biofilm formation by S. aureus MRSA, up to 391 µg/mL of product.

Subsequently, 6 doses of the **TEST** mixture ranging from 8000 µg/mL to 40 µg/mL were used to treat the biofilm already formed by S. aureus MRSA, and the biofilm removal capacity of the TEST product was then evaluated (Figure 3).

**Figure 3** shows treatment with 8000, 4000 or 800 µg/mL of **TEST** mixture has a marked effect on the biofilm of the MRSA strain. Damage to the biofilm by the action of the **TEST** mixture does not allow pathogenic activities to take place within the biofilm.

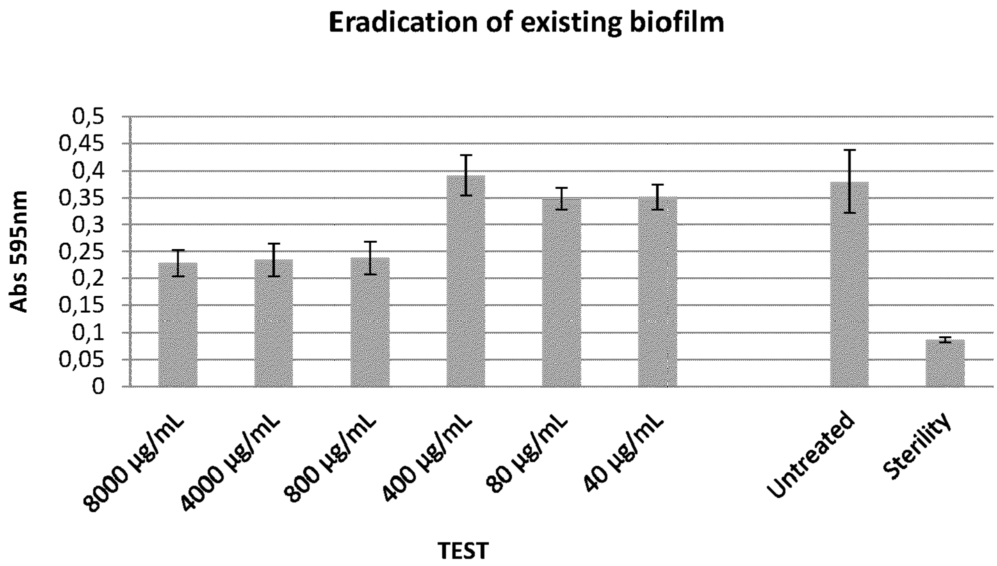


(Figure 3)

<u>**Conclusions of the experimental tests**</u>

The research evaluated the antimicrobial activity of methicillin-resistant S. aureus (DSM 46320), both in its planktonic form, i.e. free and unattached, and in the form of biofilms. It was demonstrated for the first time that the **TEST** mixture inhibited the planktonic growth of the MRSA strain, enabling an 8-fold increase in the strain's sensitivity to

methicillin. The **TEST** mixture totally inhibits biofilm formation by S. aureus DSM 46320, and destroys the existing biofilm structure. Damage to the biofilm by the action of the **TEST** mixture does not allow pathogenic activities to take place within the biofilm.

The invention thus conceived is susceptible to many modifications and variants, all falling within the same inventive concept.

Moreover, all details can be replaced by other technically equivalent elements.

In practice, the materials used, as well as the contingent shapes and sizes, can be whatever according to the requirements without for this reason departing from the scope of protection of the following claims.

## Claims

**1.** A mixture comprising:

- at least one organic or inorganic acid with bactericidal and/or bacteriostatic properties,
- at least one essential oil with anti-inflammatory and antioxidant properties,
- at least one fatty acid,
- at least one plant extract, and
- at least one carrier substance.

**2.** The mixture according to claim 1, wherein said at least one organic or inorganic acid is selected from the group consisting of: citric acid, sorbic acid, malic acid, orthophosphoric acid, fumaric acid, benzoic acid, acetic acid, butyric acid, formic acid and lactic acid, in free form and/or as salts.

**3.** The mixture according to claim 1, wherein said at least one essential oil is selected from the group consisting of: alcohols, aldehydes, ketones, esters, phenols, sesquiterpenes and terpenes.

**4.** The mixture according to claim 1 or 3, wherein said at least one essential oil is obtained from a pure plant extract identical to the natural one.

**5.** The mixture according to claim 1, wherein said at least one fatty acid is a medium chain fatty acid in free form and/or as a monoglyceride.

**6.** The mixture according to the preceding claim, wherein the corresponding monoglyceride derivative of said fatty acid is a single-chain lipid amphiphile.

**7.** The mixture according to claim 1, wherein the plant extract is purified lignin.

**8.** The mixture according to claim 1, wherein the carrier substance consists of at least one mineral selected from the group consisting of: zeolites, bentonites, silicates, calcium salts, potassium salts and sodium chloride in the case of a dry mixture, and an oil in the case of a liquid mixture.

**9.** The mixture according to claim 1, which further comprises:

- at least one mineral salt.

**10.** The mixture according to the preceding claim, wherein the mineral salt is selected from the group consisting of Zinc (in sulphate, carbonate and oxide salt form), Iron (in sulphate, carbonate, gluconate salt form) Copper (in sulphate, carbonate salt form) Manganese (in sulphate, carbonate, chloride salt form) also in chelate form, and magnesium citrate.

Europäisches Patentamt
European Patent Office
Office européen des brevets

# EUROPEAN SEARCH REPORT

Application Number
EP 25 15 5605

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | JP 2014 057591 A (DSM IP ASSETS BV) 3 April 2014 (2014-04-03)<br>* claims 1-12 *<br>* paragraphs [0002] - [0010], [0025] - [0037], [0044] - [0057] *<br>* examples 1-5 *<br>* figures 1,2 *<br>----- | 1-10 | INV.<br>A23K20/111<br>A23K20/132<br>A23K20/158<br>A23L29/00<br>A61K31/06 |
| X | EP 2 473 063 B1 (ANITOX CORP [US]) 12 April 2017 (2017-04-12)<br>* claims 1-13 *<br>* paragraphs [0019] - [0046] *<br>* examples 1,2,3,4,7,8 *<br>----- | 1-10 | |
| X | US 2013/230609 A1 (MODAK SHANTA [US] ET AL) 5 September 2013 (2013-09-05)<br>* claims 1-19 *<br>* examples 1-4 *<br>* paragraphs [0001], [0024], [0056] - [0094] *<br>----- | 1-10 | TECHNICAL FIELDS SEARCHED (IPC) |
| X | US 2014/242198 A1 (MODAK SHANTA M [US] ET AL) 28 August 2014 (2014-08-28)<br>* claims 1-17 *<br>* examples 1-4 *<br>* paragraphs [0002], [0023] *<br>-----<br>-/-- | 1-10 | A23K<br>A61K<br>A23L |

The present search report has been drawn up for all claims

1

| Place of search | Date of completion of the search | Examiner |
|---|---|---|
| Munich | 4 June 2025 | Barac, Dominika |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 1 of 2

Europäisches Patentamt
European Patent Office
Office européen des brevets

# EUROPEAN SEARCH REPORT

Application Number
EP 25 15 5605

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | LÓPEZ-GÁLVEZ GLORIA ET AL: "Alternatives to antibiotics and trace elements (copper and zinc) to improve gut health and zootechnical parameters in piglets: A review", ANIMAL FEED SCIENCE AND TECHNOLOGY, ELSEVIER, AMSTERDAM, NL, vol. 271, 24 October 2020 (2020-10-24), XP086443435, ISSN: 0377-8401, DOI: 10.1016/J.ANIFEEDSCI.2020.114727 [retrieved on 2020-10-24] * the whole document * ----- | 1-10 | |

TECHNICAL FIELDS SEARCHED (IPC)

The present search report has been drawn up for all claims

1

| Place of search | Date of completion of the search | Examiner |
|---|---|---|
| Munich | 4 June 2025 | Barac, Dominika |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 2

## ANNEX TO THE EUROPEAN SEARCH REPORT ON EUROPEAN PATENT APPLICATION NO. EP 25 15 5605

This annex lists the patent family members relating to the patent documents cited in the above-mentioned European search report. The members are as contained in the European Patent Office EDP file on
The European Patent Office is in no way liable for these particulars which are merely given for the purpose of information.

04-06-2025

| Patent document cited in search report | Publication date | Patent family member(s) | Publication date |
|---|---|---|---|
| JP 2014057591 A | 03-04-2014 | BR PI0804911 A2 | 14-07-2009 |
| | | CN 101331915 A | 31-12-2008 |
| | | CN 102239962 A | 16-11-2011 |
| | | EP 2042041 A2 | 01-04-2009 |
| | | EP 2368440 A1 | 28-09-2011 |
| | | ES 2487642 T3 | 22-08-2014 |
| | | ES 2523667 T3 | 28-11-2014 |
| | | JP 5413943 B2 | 12-02-2014 |
| | | JP 6024052 B2 | 09-11-2016 |
| | | JP 2009011320 A | 22-01-2009 |
| | | JP 2014057591 A | 03-04-2014 |
| | | PL 2042041 T3 | 27-02-2015 |
| | | PL 2368440 T3 | 31-10-2014 |
| | | TR 200804792 A2 | 21-01-2009 |
| | | US 2009004308 A1 | 01-01-2009 |
| EP 2473063 B1 | 12-04-2017 | AU 2009351659 A1 | 12-04-2012 |
| | | CA 2769985 A1 | 03-03-2011 |
| | | CN 102480997 A | 30-05-2012 |
| | | EG 26784 A | 09-09-2014 |
| | | EP 2473063 A1 | 11-07-2012 |
| | | EP 3170392 A1 | 24-05-2017 |
| | | ES 2622875 T3 | 07-07-2017 |
| | | ES 3008807 T3 | 25-03-2025 |
| | | HU E033231 T2 | 28-11-2017 |
| | | IL 218104 A | 28-02-2017 |
| | | JP 5416841 B2 | 12-02-2014 |
| | | JP 2013502924 A | 31-01-2013 |
| | | KR 20120083358 A | 25-07-2012 |
| | | LT 2473063 T | 12-06-2017 |
| | | MY 159542 A | 13-01-2017 |
| | | PL 2473063 T3 | 31-08-2017 |
| | | PL 3170392 T3 | 07-04-2025 |
| | | PT 2473063 T | 20-06-2017 |
| | | RU 2012109578 A | 20-10-2013 |
| | | UA 101280 C2 | 11-03-2013 |
| | | US 2012148718 A1 | 14-06-2012 |
| | | WO 2011025496 A1 | 03-03-2011 |
| | | ZA 201201501 B | 31-10-2012 |
| US 2013230609 A1 | 05-09-2013 | NONE | |
| US 2014242198 A1 | 28-08-2014 | US 2014242198 A1 | 28-08-2014 |
| | | US 2018206505 A1 | 26-07-2018 |

EPO FORM P0459

For more details about this annex : see Official Journal of the European Patent Office, No. 12/82